# EUROPÄISCHE PATENTANMELDUNG

(11) **EP 1 240 912 A1**
(43) Veröffentlichungstag der Anmeldung: **18.09.2002**
(21) Anmeldenummer: 02002438.6
(22) Anmeldetag: 01.02.2002
(51) Int. Cl.: A61M 5/14

(54) **Standgestell zum Einhängen von Beutelpaar-Filter-Einheiten**

(30) Priorität: 02.02.2001 DE 10104686
(71) Anmelder: DRK-BLUTSPENDEDIENST Nordrhein-Westfalen gGmbH, 48151 Münster (DE)
(72) Erfinder: Schmitz, Frank, 48432 Rheine (DE)
(74) Vertreter: Hoffmeister, Helmut, Dr.

(57) **Zusammenfassung**

Die Erfindung betrifft ein Standgestell 100 zum Einhängen und translatorischen Bewegen von Beutelpaar-Filter-Einheiten 1, mit einem im Kopfbereich des Standgestells angeordneten, bewegbaren Beutelträger 30, der mit Haken 20 zum Einhängen jeweils eines zu einer Beutelpaar-Filter-Einheit gehörenden Beutels 2 ausgerüstet ist, dadurch gekennzeichnet, daß der Beutelträger folgende Elemente umfaßt:
- zwei Endlos-Förderketten-Schleifen 7, 8, die quer zu ihrer Förderrichtung beabstandet angeordnet und synchron antreibbar sind,
- eine Anzahl von zwischen den Förderketten 9 in paralleler Anordnung verlaufende Trägertraversen 12, die an ihren Enden mit gegenüberliegenden Gliedern 13 beider Förderketten verbunden sind,
- wobei der Abstand des Beutelträgers 30 vom Bodenniveau entweder verstellbar ist oder so fixiert ist, daß die Trägertraversen, die sich im Förderketten-Untertrum befinden, vom Bodenniveau einen Abstand haben, der wenigstens so groß ist wie die Länge einer Beutelpaar-Filter-Einheit.

## Beschreibung

Die Erfindung betrifft Standgestell zum Einhängen und translatorischen Bewegen von Beutelpaar-Filter-Einheiten, mit einem im Kopfbereich des Standgestells angeordneten, bewegbaren Beutelträger, der mit Haken zum Einhängen jeweils eines zu einer Beutelpaar-Filter-Einheit gehörenden Beutels ausgerüstet ist.

Speziell bei der Verarbeitung von Blutprodukten ergibt sich das Problem, daß in einem Zentrifugationsprozeß von anderen Blutbestandteilen abgetrennte Erythrocyten, die sich in einer flüssigen Resuspensionslösung befinden, in einer sogenannten Beutelpaar-Filter-Einheit weiter verarbeitet werden müssen.

Die Beutelpaar-Filter-Einheit besteht aus zwei jeweils an den Enden eines Schlauchs befestigten geschlossenen Kunststoff-Beuteln, wobei in der Mitte der Schlauchstrecke eine Filterkapsel angeordnet ist, die Leukozyten über ein Filterkonzentrat ausfiltert. Derartige Beutelpaar-Filter-Einheiten sind an sich bekannt. Sie werden jeweils für eine Blutkonserve angelegt und benötigen jeweils etwa 15 bis 60 Minuten Filtrationszeit, in der die Beutelpaar-Filter-Einheit ruhig in einer vertikalen Anordnung hängen muß.

Bekannt ist es, sogenannte Karussel-Träger zu verwenden. Hierbei wird ein Träger verwendet, der im wesentlichen eine Runde Scheibe mit peripher angeordneten Haken umfaßt, an die jeweils der obere Beutel einer Beutelpaar-Filter-Einheit eingehängt wird. Nachteilig ist bei dem Stand der Technik, daß nur relativ wenig Beutel, ca. 20, maximal aufgehängt werden können. Weiterhin ist nachteilig, daß Anfang und Ende des jeweiligen Durchganges mehrerer Beutel sehr schwer zu markieren ist, so daß es immer wieder zu Unter- und Überschreiten der Filterzeiten kommt.

Bekannt ist aus der DE 89 08 071 U1 ein verfahrbarer Ständer zur Aufnahme von Infusionsgeräten, der als Rollenwagen mit einem Stützenpaar und starren Querstreben zwischen den Stützen gestaltet ist. An den Querstreben können mehrere Infusionsgeräte, insbesondere Behälter mit zu infundierenden Flüssigkeiten, aufgehängt werden. Nachteilig ist, dass die verfahrbaren Ständer nur eine sehr begrenzte Anzahl von Beutelpaar-Filter-Einheiten aufnehmen können.

Weiterhin ist aus EP 1 048 395 A2 eine Anordnung zum Umfüllen von in Beutel vorliegendem Spenderblut bekannt, bei der der Beutel an einer Trageeinrichtung aufzuhängen ist, so daß eine Verbindungsleitung mit Filter frei nach unten hängen kann und zu einem Auffangbeutel führt, der im Fußbereich der Anordnung in einer Abpreßstation steht. Die Aufnahmebeutel hängen unbeweglich an Bügeln und müssen jeweils genau markiert sein, damit erkannt wird, welche Beutel-Position nach Ablauf einer bestimmten Zeit neu beschickt werden kann.

Es stellt sich die Aufgabe, ein speziell zum Einhängen und translatorischen Bewegen von Beutelpaar-Filter-Einheiten ausgestattetes Standgestell anzugeben, das im Prinzip eine beliebige Anzahl von Filter-Einheiten aufnehmen kann, bei dem zuverlässig innerhalb der translatorischen Bewegung, die ein Vorrücken von einer Anfangs- in eine Endposition bedeutet, eine Filtrierzeit abgemessen werden kann, ohne daß es zu Unter- und Überschreitungen von Filtrierzeiten kommt.

Diese Aufgabe wird gelöst bei einem Standgestell der eingangs genannten Art, welches einen Beutelträger besitzt, der folgende Elemente umfaßt:
- zwei Endlos-Förderketten-Schleifen mit Förderketten, die quer zu ihrer Förderrichtung beabstandet angeordnet und synchron antreibbar sind,
- eine Anzahl von zwischen den Förderketten in paralleler Anordnung verlaufenden Trägertraversen, die an ihren Enden mit gegenüberliegenden Gliedern beider Förderketten verbunden sind,
- wobei der Abstand des Beutelträgers vom Bodenniveau entweder verstellbar ist oder so fixiert ist, daß die Trägertraversen, die sich im Förderketten-Untertrum befinden, vom Bodenniveau einen Abstand haben, der wenigstens so groß ist wie die Länge einer Beutelpaar-Filter-Einheit.

Das technische Merkmal "Förderketten" weist auf eine vorteilhafte Ausführungsform hin, nämlich die Verwendung von Ketten als Förderelement. Es soll aber nicht ausgeschlossen sein, daß auch andere bandförmige, unter Zugkraft bewegbare Elemente, wie Riemen, Bänder, Kunststoff-Gliederketten und dergleichen verwendet werden. Der Begriff "Förderketten" ist hier lediglich als anschaulich gewählt worden.

Die Förderketten werden durch Antriebsräder, insbesondere Antriebszahnräder, angetrieben, wobei hier die beiden Antriebszahnräder auf den beiden Seiten des Beutel-Trägers auf einer gemeinsamen Welle angeordnet sind.

Die Förderketten können, was vorteilhaft ist, über ein Handrad manuell angetrieben oder über einen Elektromotor im Taktbetrieb motorisch angetrieben werden. Selbstverständlich sind auch beide Antriebsarten kombinierbar, wenn eine entsprechende Kupplungseinheit zwischen der Abtriebswelle des Elektromotors und der Förderkette vorhanden ist.

Vorzugsweise werden die Förderketten von einem Schutzgehäuse wenigstens auf der den Trägertraversen abgewandten Seite eingefaßt.

Es ergibt sich eine relativ einfache Konstruktion, wenn jede Förderkette mit ihren Antriebs- und Führungsrädern an einem Längsbalken befestigt ist, der über wenigstens einen, vorzugsweise längenverstellbaren, Standholm mit einem Fußgestell verbunden ist.

Um die Trägertraversen zu reinigen oder getrennt vom Standgestell bestücken zu können, ist es vorteilhaft, wenn diese von den Förderketten abnehmbar sind.

Die Trägertraversen sind vorzugsweise aus einem Profilmaterial hergestellt, so dass sie im Querschnitt einen nach unten zeigenden Steg umfassen, der mit Γ-förmigen Einschnitten versehen ist, die die Haken bilden.

Schließlich wird vorgeschlagen, das Trägergestell mit Fahrrollen zu versehen, so daß es innerhalb der Arbeitsräume verfahrbar ist. Es ist auch möglich, das Trägergestell mit einer auf das Fußgestell aufgestützten Tischplatte zu versehen, so daß Beschriftungen, Sichtungen und dergleichen leicht vorgenommen werden können. Auch das Ablegen und Abschneiden der gefilterten Präparate in den Beuteln ist hierdurch erleichtert.

Um zu verhindern, daß die Beutel beschädigt werden oder eine bestimmte Filtrierzeit überschreiten, wird vorgeschlagen, daß im Abnahmebereich der Beutelpaar-Filter-Einheiten am Standgestell eine ein Warn- und/oder Haltesignal auslösende Vorrichtung angeordnet wird.

Ein Ausführungsbeispiel der Erfindung ist in der Zeichnung dargestellt. Die Figuren der Zeichnung zeigen im einzelnen:
Figur 1 in perspektivischer Darstellung ein Standgestells zum Einhängen von Beutelpaar-Filter-Einheiten gemäß Erfindung;
Figur 2 einen Abschnitt einer Trägertraverse mit hakenförmigen Einschnitten.

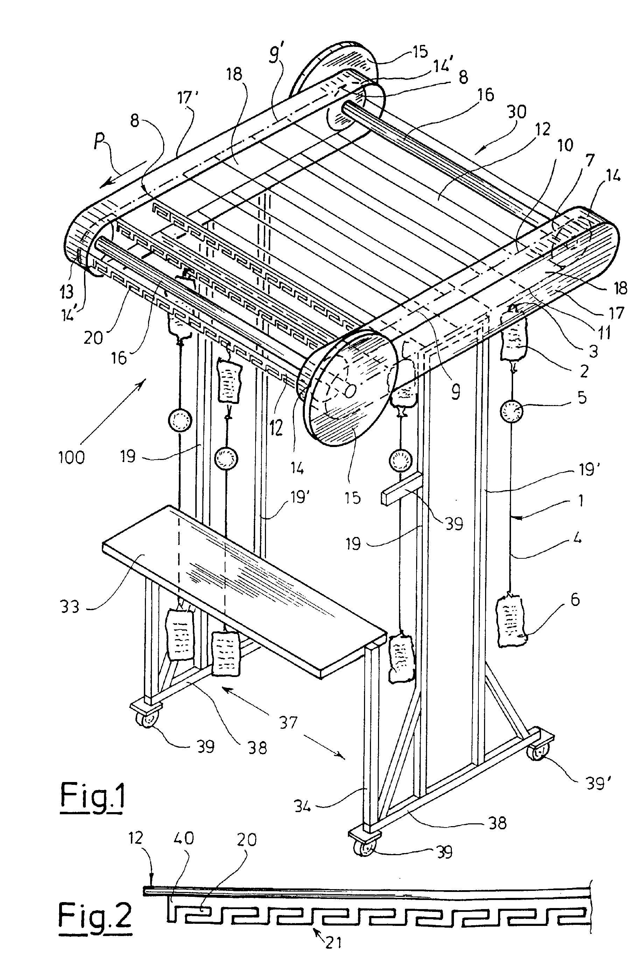

Ein in der Figur 1 dargestelltes Standgestell 100 umfaßt insbesondere einen beweglichen Beutelträger 30. Der Beutelträger ist höhenverstellbar über höhenverstellbare Standholme 19,19' getragen. Die Standholme 19,19' enden in einem Fußgestell 37, welches jeweils zwei Fußbalken 38,38' aufweist, die mit je einem Paar Rollen 39,39' unterlegt sind, so daß das gesamte Standgestell 100 verfahrbar ist. Auf dem Fußbalken 38,38' ist außerdem eine Tischplattenstütze 34 mit einer Tischplatte 33 aufgesetzt, deren Arbeitsniveau etwa in der halben Höhe der Standholme 19,19' liegt.

Wesentliches Teil des Standgestelles 100 ist der Beutelträger 30. Eingehüllt durch Schutzgehäuse 17,17' sind zwei Endlos-Förderketten-Schleifen 7,8 vorgesehen, die aus Förerkette 9 und einzelnen Kettengliedern 13 bestehen. Im vorderen und hinteren Bereich befinden sich jeweils Antriebszahnräder 14, 14', die über eine gemeinsame Welle 16 angetrieben sind, so daß beide Förderketten-Schleifen 7,8 mit ihren Förderketten 9, 9' synchron umlaufen.

Die Förderketten-Antriebsrollen 14, 14' werden manuell über ein Handrad 15 gedreht; es ist auch möglich, anstelle von Handrädern oder zusätzlich zu ihnen einen motorischen Antrieb vorzusehen, wobei diese Ausführungsform nicht dargestellt ist, aber für jeden Fachmann zu realisieren ist.

Zwischen den Förderketten 9, 9' verlaufen in paralleler Anordnung mehrere Trägertraversen 12, die an ihren Enden mit gegenüberliegenden Gliedern 13 beider Förderketten 9 verbunden sind. Die Bewegungsrichtung der Förderketten ist so, daß der Untertrum 11 sich in Richtung des Pfeiles P und der Obertrum 10 sich in Gegenrichtung bewegt.

Die vorderen und hinteren Antriebszahnräder 14, 14' und damit die beiden Endlos-Förderketten-Schleifen 7,8 sind an einem Längsbalken 18 befestigt, der auf die beiden Standholm-Paare 19,19' aufgesetzt ist.

Die Trägertraversen 12 sind in Draufsicht in Figur 2 dargestellt. Sie bestehen aus einem Winkelprofil, welches eine nach unten zeigenden Steg 40 umfaßt, wobei dieser Steg mit Γ-förmigen Einschnitten 20 versehen ist, die Haken 21 bilden.

Im Untertrum 11 der Endlos-Förderketten-Schleifen 7, 8 sind an diesen Haken 21 der Trägertraversen 12 Beutelpaar-Filter-Einheiten 1 eingehängt. Dann ist am oberen Beutel 2 eine Öse 3 vorhanden, die in den Haken 20 einzuhängen ist. Über einen Schlauch 4 ist der untere Beutel 6 mit dem oberen Beutel 2 verbunden, wobei im Mittelbereich des Schlauches 4 eine Filterkapsel 5 angeordnet ist, durch die das Erythrocyten- Gemisch aus dem oberen Beutel 2 durch den Schlauch 4 in den unteren Beutel 6 fließt.

Entsprechend den Filtrierbedingungen, die eingehalten werden müssen, dauert das Durchlaufen der Flüssigkeit vom oberen 2 zum unteren Beutel 6 etwa 20 Minuten. Geht man davon aus, daß diese Filtrierzeit genau eingehalten werden muß, so sind die zur Zeit X eingehängten Beutelpaar-Filter-Einheiten 1 zu Beginn des Untertrums 20 Minuten unterwegs. Es ist demnach erforderlich, etwa alle 3 Minuten die Förderketten um einen Trägertraversen-Abstand weiter zu bewegen. Hierzu ist das Handrad 15 vorgesehen. Am Ende der Förderstrecke können die Beutelpaar-Filter-Einheiten sehr leicht abgenommen werden, wobei durch die relative Unzugänglichkeit des Untertrums 11 verhindert wird, daß Beutel schon vor dem Ende Durchlaufen der Förderbandstrecke abgenommen werden.

Beim Aufliegen auf der Tischplatte 33 können die Beutel 6 kontrolliert, markiert und entsprechend für die Weiterverarbeitung bereitgelegt werden.

Mit Hilfe eines am Holm 19 angebrachten optischen Gerätes 36 kann außerdem festgestellt werden, daß bei der automatischen oder manuell betriebenen Bewegung Beutelpaar-Filter-Einheiten in eine bestimmte Position gelangen. Damit das Personal hierauf aufmerksam gemacht wird, kann die Vorrichtung ein Warnsignal oder ein Haltesignal auslösen.

Insgesamt ergibt sich damit eine Vorrichtung, die für den vorgenannten speziellen Zweck konzipiert eine besonders gute Lösung der im Betrieb von Blutspende-Einrichtungen sich ergebenden Probleme darstellt.

## Patentansprüche

1. Standgestell (100) zum Einhängen und translatorischen Bewegen von Beutelpaar-Filter-Einheiten (1), mit einem im Kopfbereich des Standgestells angeordneten, bewegbaren Beutelträger (30), der mit Haken (20) zum Einhängen jeweils eines zu einer Beutelpaar-Filter-Einheit (1) gehörenden Beutels (2) ausgerüstet ist, **dadurch gekennzeichnet, daß** der Beutelträger (30) folgende Elemente umfaßt:
- zwei Endlos-Förderketten-Schleifen (7,8) mit Förderketten (9,9'), die quer zu ihrer Förderrichtung beabstandet angeordnet und synchron antreibbar sind,
- eine Anzahl von zwischen den Förderketten (9,9') in paralleler Anordnung verlaufenden Trägertraversen (12), die an ihren Enden mit gegenüberliegenden Gliedern (13) beider Förderketten (9,9') verbunden sind,
- wobei der Abstand des Beutelträgers (30) vom Bodenniveau entweder verstellbar ist oder so fixiert ist, daß die Trägertraversen, die sich im Förderketten-Untertrum(11) befinden, vom Bodenniveau einen Abstand haben, der wenigstens so groß ist wie die Länge einer Beutelpaar-Filter-Einheit (1).

2. Standgestell nach Anspruch 1, **dadurch gekennzeichnet, daß** die Förderketten jeweils über ein erstes Antriebszahnrad (14) antreibbar sind, das auf einer gemeinsamen Welle (16) mit dem anderen Antriebszahnrad (14') angeordnet sind.

3. Standgestell nach Anspruch 1 oder 2, **dadurch gekennzeichnet, daß** die Förderketten-Schleifen (7,8) über ein Handrad (15) manuell oder über einen Elektromotor motorisch antreibbar sind.

4. Standgestell nach Anspruch 1 bis 3, **dadurch gekennzeichnet, daß** die Förderketten-Schleifen (7,8) von einem Schutzgehäuse (17;17') wenigstens auf der den Trägertraversen (12) abgewandten Seite eingefaßt sind.

5. Standgestell nach einem der vorhergehenden Ansprüche, **dadurch gekennzeichnet, daß** jede Förderketten-Schleife (7;8) mit ihren Antriebs- und Führungsrädern (14,14') an einem Längsbalken (18) befestigt ist, der über wenigstens einen, vorzugsweise längenverstellbaren, Standholm (19;19') mit einem Fußgestell (37) verbunden ist.

6. Standgestell nach einem der vorhergehenden Ansprüche, **dadurch gekennzeichnet, daß** die Trägertraversen (12) von den Förderketten (9) abnehmbar sind.

7. Standgestell nach einem der vorhergehenden Ansprüche, **dadurch gekennzeichnet, daß** die Trägertraversen (12) im Querschnitt einen nach unten zeigenden Steg (40) umfassen, der mit Γ-förmigen Einschnitten (20) versehen ist, die die Haken (20) bilden.

8. Standgestell nach einem der vorhergehenden Ansprüche, **dadurch gekennzeichnet, daß** es mit Fahrrollen (39) versehen ist.

9. Standgestell nach einem der vorhergehenden Ansprüche, **dadurch gekennzeichnet, daß** das Trägergestell mit einer auf das Fußgestell (37) aufgestützten Tischplatte (33) versehen ist.

10. Standgestell nach einem der vorhergehenden Ansprüche, **dadurch gekennzeichnet, daß** im Abnahmebereich der Beutelpaar-Filter-Einheiten eine ein Warn- und/oder Haltesignal auslösende Vorrichtung (36) angeordnet ist.
